# EUROPEAN PATENT APPLICATION

(11) **EP 1 548 109 A1**
(43) Date of publication of application: **29.06.2005**
(21) Application number: 03794269.5
(22) Date of filing: 05.09.2003
(51) Int. Cl.: C12N 15/09, C07H 21/02

(54) **METHOD OF AMINOACYLATING tRNA**

(30) Priority: 09.09.2002 JP 2002262301
(71) Applicant: Japan Science and Technology Agency, Kawaguchi-shi, Saitama 332-0012 (JP)
(72) Inventor: SISIDO, Masahiko, Okayama-shi, Okayama 700-0082 (JP); NINOMIYA, Keiko, Neyagawa-shi, Osaka 572-0825 (JP)
(74) Representative: Hiebl, Inge, Dr.
(86) International application number: PCT/JP2003/011391
(87) International publication number: WO 2004/022744

(57) **Abstract**

It is intended to provide a method of chemically synthesizing an aminoacyl tRNA which is completely different from the existing methods, namely, a highly efficient and practically usable method for synthesizing an aminoacyl tRNA whereby any nonnatural amino acid can be conveniently aminoacylated without a need for genetic engineering techniques and detected without resort to a radioactive isotope.

The above-described aminoacylation method comprises enclosing a tRNA and an amino acid in the vicinity of the micelle-water interface and bringing them close to each other to thereby react the same, or providing between them a peptide nucleic acid specifically binding to the tRNA as an antisense molecule and bringing them close to each other to thereby react the same.

## Description

### Technical Field

The present invention relates to a method for aminoacylating a tRNA. More specifically, the present invention relates to a method for attaching a nonnatural amino acid to a tRNA (transfer RNA), namely an aminoacylation method of a tRNA, which is essential in the introduction of nonnatural amino acids into a protein. In addition, it is a matter of course that this method can also be applied to natural amino acids.

### Background Art

The genomic research has progressed and a comprehensive proteome research is becoming practical. However, it is difficult to understand the functions of a number of proteins that are complicated in their structures. For this reason, as a method for analyzing the function of a protein, it has been becoming necessary to add an artificial function to a protein by introducing a functional amino acid into a specific position of the protein to elucidate their structures and functions.

On the other hand, it is considered necessary to produce a protein in which a nonnatural amino acid has been introduced for the purposes of a proteome analysis, creation of a protein that shows a significant performance such as a medicinal effect, or the like.

For the production of a protein containing a nonnatural amino acid, the attachment of a nonnatural amino acid to a tRNA (aminoacylation of a tRNA) is essential. In this regard, although many studies have been carried out, currently available method is very limited and complicated. This is a bottle-neck step in the development of proteins containing nonnatural amino acids.

At present, researchers all over the world have been attempting aminoacylation by a variety of methods. The most generally used method is the chemical aminoacylation developed by Hecht et al. In this method, a truncated tRNA, which is two residues shorter than usual tRNAs, is produced by a biochemical method and, at the same time, chemically aminoacylated two residues are synthesized chemically, then those are ligated with ligase, a ligating enzyme. This method is complicated and requires an advanced technique (for example, see the related art document 1). Further, the yield of aminoacyl-tRNA which can be synthesized by this method is low, and an improvement of the yield cannot be expected as much.

On the other hand, Schultz et al. have tried to introduce a nonnatural amino acid by modifying natural aminoacyl-tRNA synthetases by using genetic engineering technique (for example, see the related art document 2). This method is considered to require an advanced technique, and the yield is low. Further, there is a technical limitation for the use of nonnatural amino acids as a substrate, which have a variety of side chain structures.

Further, Suga et al. have attempted aminoacylation by a method that is different from that of Hecht et al. In his method, a functional RNA (ribozyme) evolved in vitro is produced and a tRNA is aminoacylated (for example, see the related art document 3). In this method, aminoacylation with nonnatural amino acids has not yet been successful and, further, a product cannot be obtained unless an amino acid-RNA, which is a substrate, is added 100-fold equivalent amount of a tRNA.

Yamashita et al. have reported aminoacylation using an aminoacyl-tRNA hydrolase (for example, see the related art document 4). This method is not practicable and has not been used in practice because the yield of aminoacylation is low, the stability of the enzyme is low, etc. as well as a problem with substrate specificity.

In addition, although the present inventors have presented the result of study regarding aminoacylation of a tRNA using an antisense molecule at the annual meeting of the Chemical Society of Japan previously (for example, see the related art document 5), they presented only its possibility there and, at that stage, they did not succeed in aminoacylating a tRNA, a novel and effective method for aminoacylating a tRNA was not found, or a specific operation procedure of aminoacylating a tRNA was not established.

Related art documents relevant to the invention of this application are listed below.
1. Heckler, T. G.; Chang, L. H.; Zama, Y.; Naka, T.; Chorghade, M. S.; Hecht, S. M., Biochemistry, 1984, 23, 1468-.
2. Noren, C. J.; Anthony-Cahill, S. J.; Griffith, M. C.; Schultz, P. G., Science, 1989, 244, 282-.
3. Bessho, Y.; Hodgson, D. R. -W; Suga, H., Nature Biotechnology, 2002, 20, 723-728.
4. JP-A-6-261756
5. Proceedings of the Chemical Society of Japan, vol. 79th, No. 2, pp. 873, 2001

### Disclosure of the invention

An object of the present invention is to provide a method for chemically synthesizing an aminoacyl-tRNA which is completely different from the conventional methods, an efficient and highly practical method for synthesizing an aminoacyl-tRNA, whereby any nonnatural amino acid can be conveniently aminoacylated without a need for genetic engineering techniques and can be detected without resort to a radioactive isotope (in the foregoing 4 cases (related art documents 1 to 4) described as a conventional art, a radioactive isotope is used in any case).

The present invention relates to a method for aminoacylating a tRNA characterized in that, in the production of an aminoacyl-tRNA by selectively aminoacylating a tRNA, the tRNA and an amino acid are brought close to each other to react with each other.

More specifically, the present invention relates to the foregoing aminoacylation method in which the tRNA and the amino acid are enclosed in the vicinity of a micelle-water interface and brought close to each other to react with each other. Alternatively, a peptide nucleic acid specifically and complementarily binding to a tRNA is interposed as an antisense molecule whereby both are brought close to each other to react with each other.

In addition, the present invention relates to the foregoing aminoacylation method in which the reaction with the tRNA is carried out by using a compound represented by the following formula [1]:

H-cAm-PNA-L-E-Am [1]

[wherein -cAm- represents a cationic amino acid residue or an oligopeptide residue consisted of 2 to 5 cationic amino acids, -PNA- represents a peptide nucleic acid residue, -L- represents a linker, -E- represents an active ester residue, and -Am represents an amino acid residue to be introduced in the tRNA].

Further, the present invention relates to a compound represented by the foregoing formula [1], which can be used in the foregoing aminoacylation method.

### Brief Description of the Drawings

Fig. 1 shows the structure of a peptide nucleic acid to be used for aminoacylation in a micelle system.
Fig. 2 shows reaction mechanisms of tRNA aminoacylation by antisense molecule.
Fig. 3 shows the structure of an amino acid-antisense molecule to be used in a method for aminoacylating a tRNA using an antisense molecule.
Fig. 4 shows an analytical method of aminoacylation in a binary system using an antisense molecule.
Fig. 5 shows an analytical method of aminoacylation by a peptide nucleic acid using DNA as a template (ternary aminoacylation using an antisense molecule).
Fig. 6 shows examples of a variety of nonnatural amino acids.
Fig. 7 shows the detection of aminoacylation by HPLC and TOF-MS.

### Best Mode for Carrying Out the Invention

Examples of the method in which a tRNA and an amino acid are brought close to each other to react with each other include, for example, a method in which a tRNA and an amino acid are enclosed in the vicinity of the micelle-water interface and brought close to each other to react with each other (hereinafter referred to as Method 1), a method in which a peptide nucleic acid that specifically and complementarily binds to a tRNA is interposed as an antisense molecule, whereby both are brought close to each other to react with each other (hereinafter referred to as Method 2) and the like.

In the Method 1, a method in which the carboxyl group of the amino acid is activated and enclosed in the micelle and, only the hydroxyl group region at the 3' end of the tRNA is inserted in the vicinity of the micelle-water interface is included. Through this method, the hydroxyl group at the 3' end of a tRNA and the activated carboxyl group of an amino acid are brought close to each other to react with each other (Method 1-1). Also, a method in which the carboxyl group of the amino acid is activated inside the micelle by using a condensing agent and, only the hydroxyl group region at the 3' end of the tRNA is inserted in the vicinity of the micelle-water interface is included. Through this method, the hydroxyl group at the 3' end of a tRNA and the activated carboxyl group of an amino acid are brought close to each other to react with each other (Method 1-2).

In other words, the aminoacylation reaction means a dehydration condensation reaction between the hydroxyl group at the 3' end of a tRNA (2'- and 3'- are both applicable) and the carboxyl group of an amino acid. What becomes a matter here is that the hydroxyl group of a tRNA and the carboxyl group, both of which have a low reactivity, are to be reacted without any side reaction (such as condensation between water to be used as a solvent and the carboxyl group). To achieve this, the carboxyl group of an amino acid is used after it is turned into a structure with a high reactivity (activation). As the method of activation, there are two methods; one is a method for activating an amino acid by using an organic synthetic method (that is, the foregoing Method 1-1) and the other is a method for activating an amino acid by using a condensing agent inside the micelle (that is, the foregoing Method 1-2).

Any of these methods can be used arbitrarily. Examples of the advantageous points of the Method 1-2 include that 1) an amino acid can be used without activating it, 2) because the active ester is formed in the micelle, isolation and purification procedures are not necessary as is needed with the activated amino acid, and further, even an unstable activated amino acid can be used, and the like.

Examples of the activated amino acid to be used in the Method 1-1 include the followings:
a) Ester
   Amino acid cyanomethyl ester (Nvoc-aa-OCM)
   Amino acid phenol ester (Nvoc-aa-OPhe)
b) Succinimide ester
   Amino acid succinimide ester (Nvoc-aa-OSu)
c) Thioester
   Amino acid thioester (Nvoc-aa-SE)
   Amino acid thiophenol ester (Nvoc-aa-SPhe)
d) Imidazole
   Amino acid imidazolide (Nvoc-aa-Im)
e) Acid anhydride
   Amino acid symmetrical acid anhydride ((Nvoc-aa)₂O)
   Amino acid mixed acid anhydride (Nvoc-aa-O-X)
Note) Nvoc: 6-nitroveratryl group, -aa-: amino acid residue

Examples of the condensing agent to be used in the Method 1-2 (a method for activating an amino acid by using a condensing agent inside the micelle) include the followings:
2-chloro-1,3-dimethylimidazolidium hexafluorophosphate: CIP
N,N'-carbonylimidazole: CDI
Diethylphosphorocyanidate: DEPC
Dicyclohexylcarbodiimide: DCC

In addition, with regard to the amino acid to be used in the Method 1, it is preferably used after the amino group is protected. The reason for the necessity of the introduction of the protecting group is to avoid the intermolecular reaction between the activated site of the amino acid and the amino group. As the protecting group of the amino group, any protecting group can be used as long as it can be easily removed after aminoacylation. However preferred examples include, for example, a 6-nitroveratryl group (Nvoc: it can be detached by irradiation with an ultraviolet ray of 354 nm), a pentenoyl group and the like. The former can be detached by irradiation with a UV lamp for about 10 minutes when it is used in a protein synthesis system after aminoacylation. On the other hand, the latter can be easily detached by a treatment with 10 mM iodine solution for about 10 minutes at room temperature when it is used in a protein synthesis system after aminoacylation.

The Method 1 of the present invention is usually carried out in the presence of a surfactant. As the surfactant, any one of a cationic surfactant, an anionic surfactant, a non-ionic surfactant or an amphoteric surfactant may be used. In addition, a system in which more than one surfactant have been mixed (for example, a non-ionic surfactant and a cationic surfactant) can also be used. However, in a system in which a cationic surfactant is used, an aminoacyl-tRNA can be obtained at the highest yield. Preferred specific examples of the cationic surfactant include, for example, cetyltrimethylammonium chloride (CTACl) and the like. Preferred specific examples of the anionic surfactant include, for example, sodium dodecyl sulfate (SDS). Specific examples of the amphoteric surfactant include, for example, phosphatidyl ethanolamine and the like. As the non-ionic surfactant, any one of an ether type, an ether-ester type, an ester type or a nitrogen-containing type may be used. However, preferred examples include an ether-ester type non-ionic surfactant such as polyoxyethylene sorbitan fatty acid ester. As a more specific brand name, Tween^{#}-20, 40, 60, 85 and the like are exemplified.

Alternatively, instead of using a surfactant, a system in which polyethyleneimine or a dendrimer which has a cationic group on the surface thereof can be used.

As the micelle, an O/W (oil in water) type is preferred, however, a W/O (reverse micelle) type, an oil-free system or the like can be used. Examples of the solvent which is used in the oil-free system include, for example, N-N-dimethylformamide (DMF) and the like.

Further, examples of the oil component in the O/W (oil in water) type include, for example, toluene, ethyl acetate, tetrahydrofuran (THF), and the like.

In the Method 1 of the present invention, examples of the method for inserting only the hydroxyl group region at the 3' end of the tRNA into the vicinity of the micelle-water interface include, for example, a method in which the 3' end group region is rendered hydrophobic by site-specifically and complementarily binding a peptide nucleic acid (PNA) in which a hydrophobic functional group has been introduced at the end thereof to the tRNA in such a manner that the hydrophobic group comes close to the vicinity of the 3' end of the tRNA.

Examples of the peptide nucleic acid (PNA) to be used in the Method 1 of the present invention include a peptide nucleic acid with a chain length n of 4 to 10, which is easy to synthesize and whose binding to a tRNA is easy to control.

Further, examples of the functional group to be introduced in a PNA include, for example, a cholic acid residue (with a steroid skeleton) such as lithocholic acid, an aliphatic carboxylic acid residue, which is hardly soluble in water, such as decanoic acid, an amino protecting group such as a fluorenylmethyloxycarbonyl group (Fmoc group), and the like.

As the method for introducing a hydrophobic functional group into a peptide nucleic acid through an amide bond, a method in which a solid phase synthesis is used is commonly used.

At the other end of the peptide nucleic acid (PNA) to be used in the Method 1, one or more molecules (usually two molecules, for example, LysLys or the like) of a cationic amino acid, for example, lysine or the like are usually introduced in order to increase the solubility in water and to increase the affinity for a tRNA, which is a polyanion.

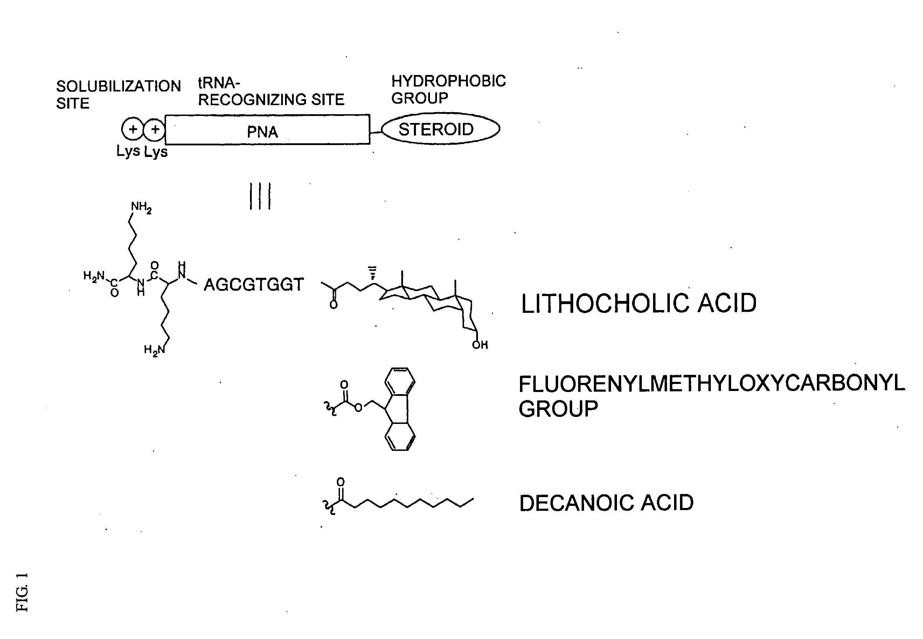

Specific examples of the structure of the PNA to be used in the Method 1 are shown in Fig. 1.

An aminoacylation reaction of the tRNA in the Method 1 of the present invention is usually carried out in the presence of a transesterification catalyst.

Examples of the transesterification catalyst to be used in the method of the present invention include a transesterification catalyst that exhibits a high catalytic activity at around a neutral pH, and more specific examples include, for example, imidazole, pyridine, dimethylaminopyridine and the like. In particular, imidazole (pKa 6.8) is preferred.

The reaction temperature in the aminoacylation reaction is usually between 0 to 50°C.

Determination of the reaction temperature depends on the chain length of the PNA to be used. In other words, the reaction is carried out at a low temperature when the PNA with a short chain length is used and at a high temperature when a PNA with a long chain length is used.

The reaction pH is in the range of a physiological condition, in other words, at around pH 7 (± 1.0).

Hereunder one example of the operation procedure of an aminoacylation method of the Method 1 is described.

### Aminoacylation operation procedure:

1. An imidazole buffer is added into a microtube and then a surfactant is added.
2. An ultrasonication treatment is carried out for 5 minutes and the mixture is micellized.
3. An amino acid active ester is added and mixed vigorously.
4. A tRNA is added and mixed, and reaction is initiated at room temperature (one hour).

After the reaction, a peptide nucleic acid is removed from the tRNA by extraction (phenol-chloroform extraction), and isolation is carried out by precipitating the tRNA by salting-out (ethanol precipitation).

Hereunder, one example of the operation procedure of an isolation and purification method which is almost common in all aminoacylation methods of the present invention will be shown.

### Operation procedure of isolation and purification method

1. To a reaction mixture (up to 20 µL), 40 µL of 1.5 M NaOAc (pH 4.5) is added.
2. Phenol is added in the same amount as the solution.
3. After the mixture is vigorously mixed, centrifugation is carried out at 4°C for 10 minutes at 15,000 rpm.
4. The upper layer (aqueous layer) is collected and transferred to another microtube. At this time, the interface should not be collected because it contains the PNA.
5. To the lower layer (phenol layer), 40 µL of 1.5 M NaOAc (pH 4.5) is added.
6. After the mixture is vigorously mixed (with a vortex), centrifugation is carried out at 40°C for 10 minutes at 15,000 rpm.
7. The upper layer (aqueous layer) is collected and transferred to another microtube. At this time, the interface should not be collected because it contains the PNA. Chloroform is added in the same amount as the collected upper layer and mixed, and then centrifugation is carried out at 4°C for 2 minutes at 15,000 rpm and the chloroform layer (lower layer) is removed.
8. 100% ethanol is added in an amount of 3 times the solution, gently mixed and left at -30°C for 30 minutes:
9. Centrifugation is carried out at 4°C for 30 minutes at 15,000 rpm.
10. The supernatant (supernatant fluid) is removed with a Pipetman. At this time, attention should be paid not to take the precipitate (nucleic acid) at the bottom.
11. 90% ethanol (200 µL) is added gently.
12. Centrifugation is carried out at 4°C for 5 minutes at 15,000 rpm.
13. The supernatant (supernatant fluid) is removed with a Pipetman. At this time, attention should be paid not to take the precipitate (nucleic acid) at the bottom.
14. Drying is carried out (under reduced pressure).

An analysis of aminoacylation may be carried out by HPLC and TOF-MS. As the method, a method in which the aminoacyl end of a tRNA, which has been aminoacylated by an antisense molecule, is digested into a mononucleotide with nuclease S1 is preferred.

If the tRNA has been aminoacylated, an aminoacyl-AMP can be detected.

Subsequently, the method in which a peptide nucleic acid that specifically and complementarily binds to a tRNA is interposed as an antisense molecule, whereby the tRNA and the amino acid are brought close to each other to react with each other (Method 2) will be described.

In order to react the hydroxyl group at the 3' end of a tRNA and an amino acid, by using a peptide nucleic acid (PNA) having a complementary sequence to the tRNA as an antisense molecule, and binding an amino acid activated ester to the end of the PNA in advance (a linker or the like is also used as needed), this amino acid-bound PNA is complementarily bound to the tRNA in such a manner that the amino acid is brought close to the 3' hydroxyl group of the tRNA. In this way, the present inventors found out that, by carrying out a transesterification reaction, the 3' hydroxyl group is selectively aminoacylated.

In addition, it was also confirmed that, by using DNA together as an antisense molecule, the foregoing amino acid-bound PNA is complementarily bound to this DNA and the DNA is also complementarily bound to the tRNA in such a manner that the amino acid is brought close to the 3' hydroxyl group, which is subjected to transesterification in the same manner, whereby the 3' hydroxyl group can be aminoacylated.

For the sake of convenience, the former is referred to as a binary system and the latter is referred to as a ternary system.

In either system, the antisense molecule is detached after the reaction.

The distinction of each system is in the point that a different reaction terminator is used; in the case of the former (binary system), an antisense molecule that forms a complementary pair with the antisense molecule is used, and in the case of the latter (ternary system), temperature is raised (for example, 25°C).

In addition, it is in the point that, in the case of the former, because it does not use DNA, it has a wider flexibility in the environment responsiveness to a reaction condition (PNA is more stable) and in the case of the latter, it can be applied to wider types of nonnatural amino acids.

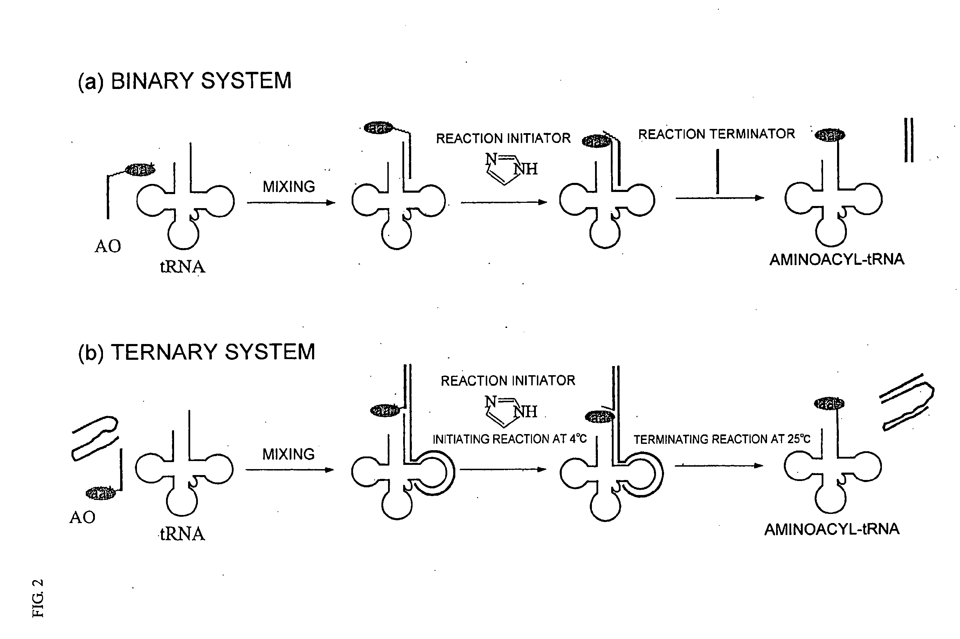

The reaction mechanisms of aminoacylation are shown in Fig. 2.

An antisense molecule (referred to as AO) 1) specifically binds to the nucleotide sequence of a tRNA by mixing, 2) expresses an aminoacylating activity by adding a reaction initiator, and 3) dissociates from the tRNA by adding a reaction terminator. A target tRNA is aminoacylated with these three steps under the perfect control.

Hereunder, the structure of an antisense molecule to be used in the binary or ternary system will be explained in the order of events.

### (1) With regard to an antisense molecule to be used in the binary system

A preferred structure of an antisense molecule (AO):

For a PNA, as a sequence binding to a tRNA, a sequence with a chain length of 4 to 10 is preferred (within a range of chain length which is easy to synthesize and whose binding to a tRNA is easy to control). Preferred examples include, for example, AAGCGTGGT with a chain length n of 9, AGCGTGGT with a chain length n of 8, GCGTGGT with a chain length n of 7, CGTGGT with a chain length n of 6 and the like, as a specific example.

In order to increase the solubility in water and to increase the affinity for a tRNA which is a polyanion, it is preferred that one or more molecules (usually two molecules) of a cationic amino acid, for example, lysine or the like, is introduced at the end. Further, in order to bring an active ester and the hydroxyl group of a tRNA close to each other, it is preferred to use a linker.

Examples of the linker include, for example, -CH₂CH₂CH₂-, -CH₂CH₂CH₂CH₂-, -CH₂OCH₂CH₂OCH₂CH₂-, or a group represented by the following formula: 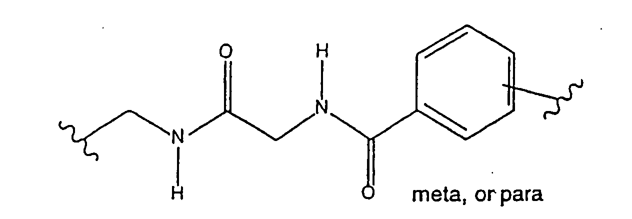 (abbreviated as abZ) and the like.

In abZ shown above, because there is a conjugated system between the peptide bond and the phenyl ring, a pi-electron system interaction with the conjugated system of the base pair of PNA and tRNA is expected, and as a result, it is possible to aim at bringing the reaction sites close to each other.

The method of active esterification may be carried out by a solid-phase synthesis.

One example of the method for synthesizing an amino acid-antisense molecule (aa-PNA) will be shown below.

A PNA monomer (Applied Biosystems Co.) is extended in accordance with a sequence to be synthesized on Fmoc-SAL-PEG-Resin (WATANABE chemical).

As the condensing agent, for example, o-(7-azobenzotriazole-1-yl)1,1,3,3-tetramethyluronium hexafluorophosphate: HATU (WATANABE Chemical), diisopropylethylamine: DIEA (WATANABE Chemical) or the like can be used.

As the solvent, for example, dimethylacetoamide: DMAA (WATANABE Chemical) or the like can be preferably used.

Subsequently, a linker, an active ester, an amino acid are sequentially condensed, whereby synthesis is carried out. After the synthesis, a target product can be obtained by cutting out with trifluoroacetic acid (TFA), and purifying by HPLC.

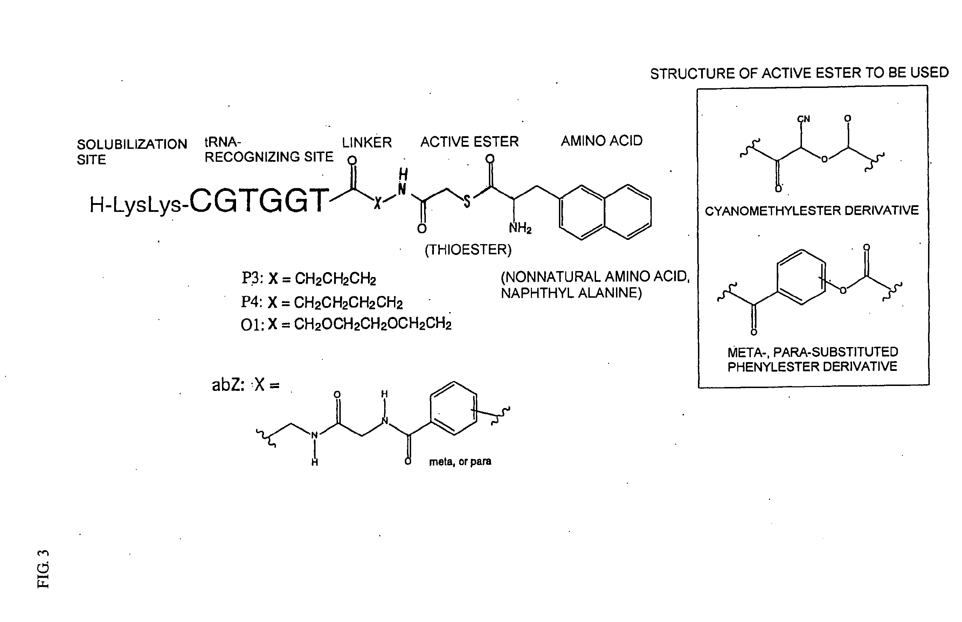

As the active ester, for example, a thioester derivative, cyanomethylester derivative, succinimide ester, m- (or p-) substituted phenylester derivative or the like is preferably used (the structural formulae are shown in Fig. 3).

Preferred examples of the amino acid-antisense molecule (aa-PNA) to be used in the present invention include, for example, a compound represented by the following formula [1]:

H-cAm-PNA-L-E-Am [1]

[wherein -cAm- represents a cationic amino acid residue or an oligopeptide residue consisted of 2 to 5 cationic amino acids, -PNA- represents a peptide nucleic acid residue, -L- represents a linker, -E- represents an active ester residue, and -Am represents an amino acid residue to be introduced in the tRNA].

The details of each component are as already described.

In addition, with regard to the amino acid to be introduced in the tRNA, either a natural or a nonnatural amino acid may be used, however, it is more effective to apply to a nonnatural amino acid which had been so far assumed to be very difficult to attach to a tRNA (aminoacylation of a tRNA) .

Examples of the nonnatural amino acid to be applicable include various amino acids, for example, a fluorescent amino acid, electron-donating amino acid, electron- accepting amino acid, photodegrading amino acid, and photoisomerizing amino acid and the like.

The structural formula of a preferred example of the amino acid-antisense molecule (aa-PNA) to be used in the present invention is shown in Fig. 3.

### (2) With regard to an antisense molecule to be used in the ternary system

As the antisense molecule to be used together with a peptide nucleic acid (PNA), DNA is preferred. RNA is expensive (about 10 times more expensive than DNA), its stability is lower than that of DNA, and its binding to a tRNA is weaker than that of DNA, therefore RNA is not preferred. Further, with regard to a PNA, there are problems that, when the chain length of a PNA becomes long, synthesis of the PNA is not easy, the control by temperature is difficult because its binding to a tRNA is too strong, and the like.

As a preferred chain length of DNA, DNA with a chain length of (7 to 12) + (0 to 2) + (10 to 23) is exemplified.

(7 to 12): the sequence binding (hybridizing) to a PNA. It is restricted to the length which is easy to control.

(0 to 2): the spacer sequence corresponding to the length of an active ester site.

(10 to 23): the sequence binding to a tRNA. It is restricted to the length which is easy to control.

On the other hand, as the PNA of an antisense molecule (AO), a sequence (with a chain length of 7 to 12) that binds to DNA but does not bind to a tRNA is preferred.

When it is too long, it becomes difficult to dissociate the binding by temperature because the binding to DNA is too strong. On the other hand, when it is too short, aminoacylation does not occur because the binding to DNA is too weak.

The structure and the synthetic method of a preferred amino acid-antisense molecule (aa-PNA) to be used in the ternary system follow those in the binary system.

Subsequently, the reaction condition of the aminoacylation method of the present invention using the antisense molecule will be described.

With regard to the reaction temperature, the reaction is usually carried out at 0 to 20°C where aminoacylation activity exists. It is inactive at 37°C. It is 45°C where a binding is completely broken, however, the reaction efficiency decreases if each component does not firmly bind to each other.

The determination of the reaction temperature depends on the chain length of a PNA to be used. In other words, the reaction is carried out at a low temperature when a PNA with a short chain length is used and at a high temperature when a PNA with a long chain length is used. The reaction pH is in the range of a physiological condition, in other words, the reaction is carried out at around pH 7 (± 1.0).

The reaction is usually carried out in the presence of a reaction initiator (catalyst). Examples of the reaction initiator to be used include a transesterification catalyst exhibiting a high catalytic activity at around a neutral pH, and specific examples include, for example, imidazole, pyridine, dimethylaminopyridine and the like, and in particular, imidazole (pKa 6.8) is preferred.

In addition, in this reaction, a sodium acetate buffer or the like can be used effectively as a reaction initiator.

The termination of the reaction is carried out by using a reaction terminator in the binary system.

As the reaction terminator, a PNA oligomer (cPNA) that forms a complementary pair with an aa-PNA is preferably used. It is known that the formation of double-stranded chain is stable in the order of PNA-PNA > PNA-RNA > PNA-DNA. Accordingly, the cPNA can separate the aa-PNA, which has been bound to a tRNA, from the tRNA and can form a PNA double-stranded chain. Accordingly, the reaction can be terminated.

In the ternary system, the termination of the reaction is carried out by raising the temperature. The temperature point to be raised is usually 25°C or higher.

Hereunder, one example of the operation procedure of an aminoacylation method in the Method 2 will be shown.

### Aminoacylation operation procedure:

In the case of the binary system
1. A buffer is added into a microtube, and then a tRNA and an aa-PNA are added.
2. The microtube is let stand on ice for 5 minutes, whereby the tRNA and the aa-PNA are bound to each other.
3. A reaction initiator (imidazole buffer) is added and reaction is carried out on ice (usually for 1 to 2 hours).

After the reaction is carried out for 1 to 2 hours, the reaction is terminated by adding a reaction terminator, cPNA.

On the other hand, the summary of the case of the ternary system is as follows.
1. A buffer is added into a microtube, and then a tRNA and a template DNA are added and the mixture is mixed with a vortex, spun down to have them hybridized.
2. An aa-PNA is added to the microtube and the mixture is mixed with a vortex, spun down and let stand on ice for 5 minutes.
3. A reaction initiator (imidazole buffer) is added and reaction is carried out on ice (usually for 2 hours).

After the reaction is carried out for 2 hours, the ice bath is removed and the reaction is terminated by raising the temperature to 25°C.

In either case, with regard to the isolation and purification method after the reaction, it is enough to carry out the procedure in accordance with the isolation and purification method described in the part of the method in the micelle system (Method 1).

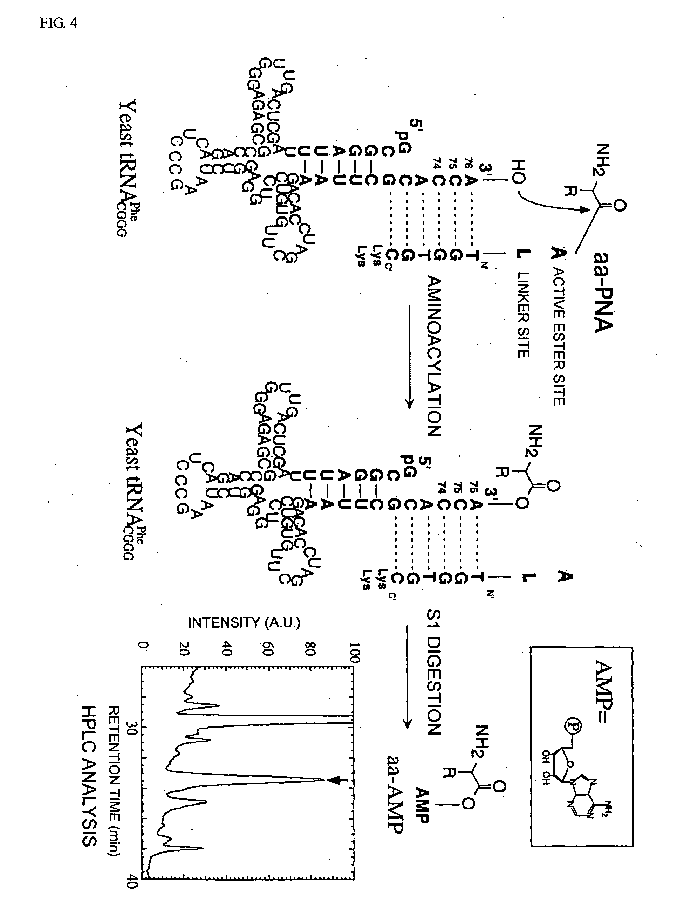

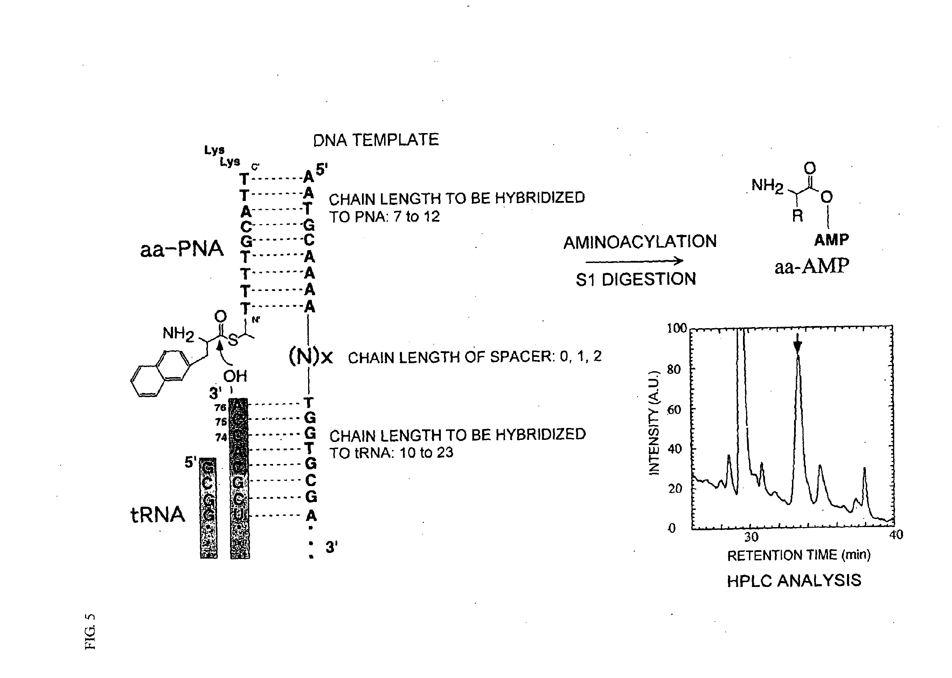

The results of analyzing and illustrating each of the aminoacylation in the binary system and the aminoacylation in the ternary system (aminoacylation by a PNA using DNA as a template) are shown in Fig. 4 and Fig. 5, respectively.

According to the method of the present invention (in either of the Method 1 or the Method 2), it is possible to introduce a variety of amino acids, either natural or nonnatural, in a tRNA efficiently and to carry out aminoacylation. Particularly, it is more effective to introduce, in a tRNA, a nonnatural amino acid which could not be bound to a tRNA with a conventional aminoacyl-tRNA synthetase, for example, a nonnatural amino acid with a structure that is significantly different from that of a natural amino acid, an amino acid with a fluorescent property, an amino acid with a photoisomerization ability, an amino acid with an oxdating ability, an amino acid with a medicinal effect against HIV virus and the like.

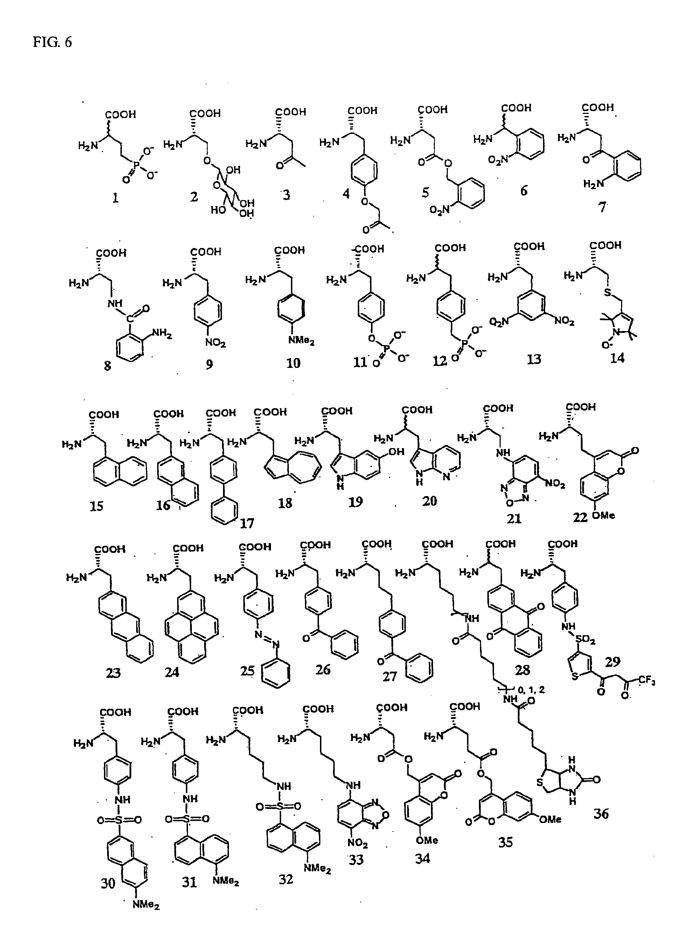

Fig. 6 shows examples of a nonnatural amino acid which can be introduced in a tRNA by the method of the present invention.

In Fig. 6, the amino acids of 1, 8, 21 to 23, and 30 to 35 are an amino acid with a fluorescent property, the amino acid of 25 is an amino acid with a photoisomerization ability, the amino acid of 28 is an amino acid with an oxdating ability, and the amino acid of 15 is an amino acid with a medicinal effect against HIV virus.

The contents described in the specification of JP-A-2002-262301 are all incorporated in this description.

### Examples

Hereunder, the present invention will be explained in more detail with reference to the Examples, however, the present invention is by no means limited to these Examples.

### Example 1 (Aminoacylation of tRNA using non-ionic micelle)

Using 2-naphthyl alanine having a naphthyl group at the side chain which has been subjected to an active esterification as a nonnatural amino acid, aminoacylation was carried out by reacting this 2-naphthyl alanine with a tRNA in the presence of a peptide nucleic acid (PNA). Incidentally, as the PNA, CGTGGT with a chain length n of 6, in which an Fmoc group and a LysLys group had been introduced as a hydrophobic group and a solubilization site, respectively, was used.

### <Composition of reaction solution for aminoacylation>

| | |
|---|---|
| 0.5 M Tween ^{#}20 | 5 µL (final concentration = 250 mM) |
| Nvoc-napAla-OCM (1 M/toluene) | 1 µL (final concentration = 100 mM) |
| 4 M imidazole-AcOH, pH 6.5 | 1 µL (final concentration = 400 mM) |
| 0.4 mM tRNA | 2 µL (fmal concentration = 80 µM) |
| 0.8 mM Fmoc-PNA | 1 µL (final concentration = 80 µM) |
| | 10 µL |

### [Operation procedure]

The imidazole buffer, then Tween ^{#}20 were added, and the mixture was micellized by ultrasonic wave. Then, the activated amino acid Nvoc-napAla-OCM was added and mixed by pipetting. After confirming that the mixture became clear, a reaction was initiated by adding the tRNA and the Fmoc-PNA.

After the mixture was reacted for 1 hour, the tRNA purification was carried out by a phenol-chloroform treatment and ethanol precipitation according to the operation procedure of the isolation and purification method described in the section [0016]. The yield was 14%.

### <Nuclease treatment and HPLC analysis>

To the obtained compound, 10 µL of a nuclease S1 buffer (pH 4.5) was added and 0.5 µL of nuclease S1 (100 units/µL) was pipetted thereto. After being incubated at 37°C for 10 minutes, the mixture was analyzed by HPLC.

### <HPLC analysis condition>

0.1 M ammonium acetate/MeOH, flow rate = 0.6 ml/minute, CI8 column, 2% up (0 to 100%).
Detection wavelength: 260 nm (UV-vis detector), 285/330 nm (Fluorescence detector).

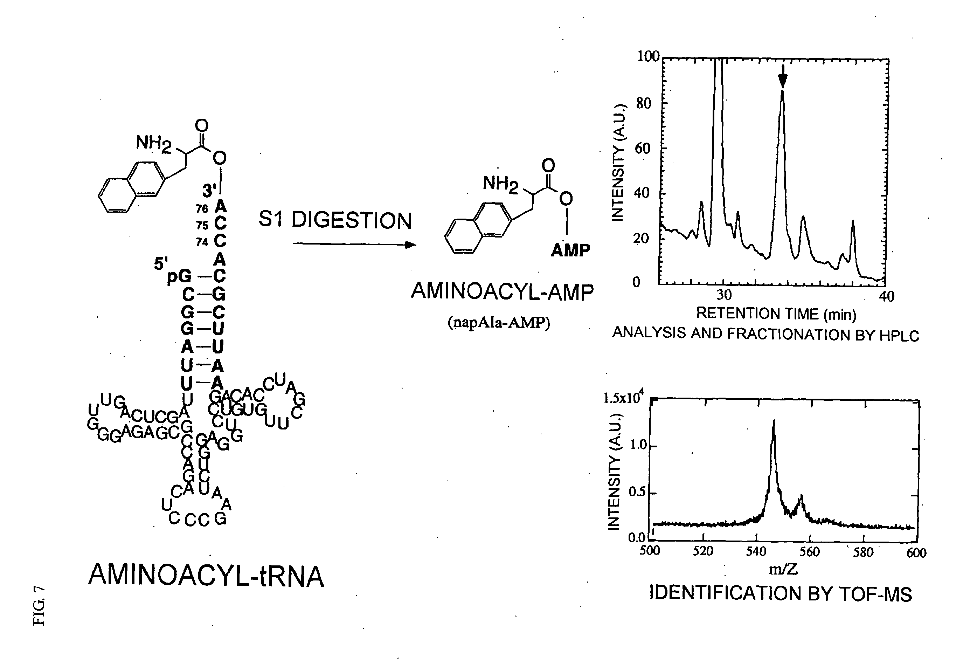

As a result, a peak which was considered to be naphthylalanyl AMP was detected by HPLC. Then, the molecular weight of this peak was analyzed by TOF-MS, and as a result, it could be identified that the peak corresponds to the target product (see Fig. 7).

### Example 2 (Aminoacylation oftRNA using non-ionic micelle)

A reaction and a post-treatment were carried out in exactly the same manner as in Example 1 except for changing the composition of the reaction solution for aminoacylation as described below. The obtained product was analyzed in the same manner as in Example 1, whereby a similar result to that of Example 1 was obtained. The yield was 13%.

### <Composition of reaction solution for aminoacylation>

| | |
|---|---|
| 0.5 M Tween ^{#}40 | 5 µL (final concentration = 250 mM) |
| Nvoc-napAla-OCM (1 M/toluene) | 1 µL (final concentration = 100 mM) |
| 4 M imidazole-AcOH, pH 6.5 | 1 µL (final concentration = 400 mM) |
| 0.4 mM tRNA | 2 µL (fmal concentration = 80 µM) |
| 0.8 mM Fmoc-PNA | 1 µL (final concentration = 80 µM) |
| | 10 µL |

### Example 3 (Aminoacylation of tRNA using cationic micelle)

### <Composition of reaction solution for aminoacylation>

| | |
|---|---|
| 20 mM CTACl/100 mM imidazole (pH7.5) | 18 µL |
| 100 mM Pentenoyl-napAla-OCM/DMF | 1 µL |
| 200 µM tRNA | 1 µL |
| | 20 µL |

The foregoing reaction solution was mixed for 10 minutes using a vortex mixer. In addition, the solution on the sidewall was brought down by centrifugation with a bench centrifuge every time when mixing is carried out for 20 to 40 seconds. To the reaction solution, 60 µL of 1.5 M AcOK was added, and 80 µL of phenol/chloroform (1:1) was further added and the mixture was mixed with a vortex mixer for several seconds (it turned out to be a white suspension). Then, the mixture was centrifuged at 15,000 rpm for several seconds at 4°C and the supernatant was recovered. To the recovered supernatant, 80 µL of CHCl₃/i-PrOH (24:1) was added and the mixture was mixed with a vortex mixer for several seconds (it turned out to be a white suspension). In the same manner as above, the mixture was centrifuged at 15,000 rpm for several seconds at 4°C and the supernatant was recovered. To the supernatant, 360 µL of ethanol was added and mixed gently, and after being left at -30°C for 1 hour, the mixture was centrifuged at 15,000 rpm for 30 minutes at 4°C and the supernatant was removed.

200 mL of 70% ethanol (-30°C) was added thereto, and the mixture was centrifuged at 15,000 rpm for several seconds at 4°C and the supernatant was removed. Lastly, the remaining product was dried under reduced pressure for about 15 minutes and it was confirmed that ethanol was completely evaporated. The dried sample was stored at -30°C until use.

To the dried sample, 10 mL of 10x nuclease S1 buffer and 1 mL of nuclease S1 were added, and after the mixture was incubated at 37°C for 15 minutes, an HPLC analysis was carried out in the same manner as in Example 1, whereby a similar result to that of Example 1 was obtained. The yield was 50%.

### Example 4 (Aminoacylation of tRNA using anionic micelle)

### <Composition of reaction solution for aminoacylation>

| | |
|---|---|
| 10 mM SDS/milliQ (ultra pure water) | 17 µL |
| 200 µM tRNA | 1 µL |
| 100 mM Pentenoyl-napAla-OCM/THF | 1 µL |
| 100 mM imidazole buffer (pH 7.5) | 1 µL |
| | 20 µL |

The foregoing reaction solution was mixed and reacted for 5 hours at room temperature. Then, 60 µL of 1.5 M AcOK was added to the reaction solution and extraction was carried out with 80 µL of phenol/chloroform and further extraction was carried out with 80 µL of chloroform.

Then, 360 µL of ethanol was added and the mixture was gently mixed, and after being let stand at -30°C for 1 hour, the mixture was centrifuged at 15,000 rpm for 30 minutes at 4°C and the supernatant was removed. 200 µL of 70% ethanol (-30°C) was added thereto, and the mixture was centrifuged at 15,000 rpm for 5 seconds at 4°C. After the supernatant was removed, the remaining product was dried under reduced pressure. 10 µL of 10x nuclease S buffer and 1 µL of nuclease S1 were added thereto, and after the mixture was incubated at 37°C for 15 minutes, an HPLC analysis was carried out in the same manner as in Example 1, whereby a similar result to that of Example 1 was obtained. The yield was 7%.

### Example 5 [Aminoacylation of tRNA using antisense molecule (binary system)]

2-naphthyl alanine, which has a naphthyl group at the side chain, was selected as a nonnatural amino acid, and by using an aa-PNA in which this 2-naphthyl alanine had been introduced into an antisense molecule, aminoacylation of a tRNA was carried out. Incidentally, the used aa-PNA was the one whose linker is the foregoing abZ (para form) in the structural formula of the aa-PNA shown in Fig.3 described above.

### <Composition of reaction solution for aminoacylation>

| | |
|---|---|
| aa-PNA (1 mM) | 0.5 µL |
| tRNA (500 µM) | 1 µL |
| 1 mM imidazole | 1 µL |
| (500 mM or 1 M) imidazole | 1 µL |
| Reaction terminator (mLM) | 0.5 µL |
| | 4 µL |

### <Operation procedure>

The aa-PNA, tRNA and 1 mM imidazole (pH 7.0) were mixed and let stand at 4°C for 5 minutes. Then, 1 M (or 500 mM) imidazole (pH 7.0) was added and aminoacylation was carried out by letting the mixture stand at room temperature for 2 hours. A cPNA, a reaction terminator, and 4 µL of a nuclease S1 buffer were added and a double- stranded PNA was formed by incubating the mixture at 37°C for 1 minute. The mixture was quickly chilled at 5°C, then 1 µL of 100-fold dilution of nuclease S1 (100 units/µL) was added and the mixture was incubated at 37°C for 15 minutes.

After the reaction, a post-treatment was carried out in the same manner as in Example 1 and the obtained product was analyzed in the same manner as in Example 1, whereby a similar result to that of Example 1 was obtained. The yield was about 5%.

### Example 6 [Aminoacylation of tRNA using antisense molecule (binary system)]

A reaction was carried out using an aa-PNA with a chain length of 9 (AAGCGTGGT) instead of using the aa-PNA with a chain length of 6 described in Fig. 3 as an aa-PNA in Example 5.

### <Composition of reaction solution for aminoacylation>

| | |
|---|---|
| aa-PNA (400 µM) | 1 µL |
| tRNA (200 µM) | 2 µL |
| 1 mM phosphate buffer | 2 µL |
| sodium phosphate | 3 µL |
| milliQ (ultra pure water) | 2 µL |
| | 10 µL |

### Reaction terminator

| | |
|---|---|
| 400 µM cPNA | 1 µL |

### <Operation procedure>

The phosphate buffer (1 mM), milliQ and tRNA were mixed, and incubated at 80°C for 2 minutes, then the aa-PNA was added and hybridization was carried out by letting the mixture stand at 5°C for 2 minutes.

Then, aminoacylation was carried out by adding sodium phosphate.

The cPNA, which is a reaction terminator, and 9 µL of 300 mM AcOK (pH 4.5) were added, and after the reaction was terminated, a phenol/chloroform treatment and ethanol precipitation were carried out.

The precipitate was dissolved in 10 µL of MilliQ and 9 µL of 10x nuclease S1 buffer and 1 µL of nuclease S1 were added and the mixture was incubated at 37°C for 15 minutes.

After the reaction, a post-treatment was carried out in the same manner as in Example 1 and the obtained product was analyzed in the same manner as in Example 1, whereby a similar result to that of Example 1 was obtained. The yield was 19%.

### Example 7 [Aminoacylation of tRNA using antisense molecule (ternary system)]

Using the same aa-PNA as used in Example 3, and using DNA 14 (DNA with a chain length of 14, which binds to an RNA) as a template to bring a PNA and a tRNA close to each other, aminoacylation of the tRNA was carried out.
(1) Hybridization of tRNA and template DNA
The solutions described below were mixed by pipetting in a microtube, mixed with a vortex and spun down.

| | |
|---|---|
| tRNA (500 µM/Q) | 25 µL (final concentration = 357 µM) |
| DNA 14 (0.86 mM/Q) | 7.26 µL (final concentration = 178 µM) |
| 100 mM Tris buffer pH 6.8 | 1.75 µL (final concentration = 5 mM) |
| milliQ (ultra pure water) | 0.98 µL |
| | 35 µL |

After this solution was incubated at 80°C for 10 minutes, it was cooled down to room temperature in a PCR machine.
(2) Then, a 1.6-mL microtube was provided and 13.5 µL of the foregoing hybridization solution was added and the microtube was let stand on ice.
(3) An aa-PNA/10 mM Tris buffer pH 6.8 was prepared on ice.
Then, in the microtube, the solutions described below were mixed by pipetting, mixed with a vortex and spun down.

| | |
|---|---|
| aa-PNA (1.5 mM/DMSO) | 0.52 µL (open after it becomes room temperature) |
| milliQ (ultra pure water) | 1.56 µL |
| | 2.08 µL |

(4) To the microtube in the foregoing (2), 0.5 µL of the aa-PNA prepared in the foregoing (3) was added each and pipetting, mixing with a vortex and spin-down were carried out and the mixture was let stand on ice for 5 minutes.
(5) To the foregoing microtube, 1.93 µL of 4 M imidazole- AcOH buffer pH 6.5 was added and a reaction was initiated on ice. The reaction time was set to 2 hours.
(6) After the reaction, a post-treatment was carried out in the same manner as in Example 1 and the obtained product was analyzed in the same manner as in Example 1, whereby a similar result to that of Example 1 was obtained. The yield was 6%.

Further, the yield was improved to 9% when a sodium acetate buffer was used instead of the imidazole-AcOH buffer in the foregoing (5).

### Example 8 [Aminoacylation of tRNA using antisense molecule (ternary system)]

A reaction and a post-treatment were carried out in exactly the same manner as in Example 4 except for using 6.79 µL of DNA 23 (DNA with a chain length of 23, which binds to an RNA) (0.92 mM/Q) (final concentration = 178 µM) as a template and 1.46 µL of milliQ (ultra pure water) instead of 7.26 µL of DNA 14 (0.86 mM/Q) (final concentration = 178 µM) and 0.98 µL of milliQ in Example 4, and the obtained product was analyzed in the same manner as in Example 4, whereby a similar result to that of Example 4 was obtained. The yield was 5%.

### Example 9 (Aminoacylation of tRNA using polyethylene imine)

### <Composition of reaction solution for aminoacylation>

| | |
|---|---|
| polyethylene imine (1.09 mg)/milliQ | 17 µL |
| tRNA | 1 µL |
| 0.1 M Pentenoyl-napAla-OCM/DMF | 1 µL |
| 100 mM imidazole (pH 7.5) | 1 µL |
| | 20 µL |

The foregoing reaction solution was mixed and reacted for 3.5 hours at room temperature. Then, to the reaction solution, 70 µL of 1.5 M AcOK was added, and an equal volume of phenol/chloroform was added and extraction was carried out, and further, an equal volume of chloroform was added and extraction was carried out. Then, 360 µL of ethanol was added and mixed gently, and after being let stand at -30°C for 1 hour, the mixture was centrifuged at 15,000 rpm for 30 minutes at 4°C and the supernatant was removed. 200 µL of 70% ethanol (-30°C) was added thereto, and the mixture was centrifuged at 15,000 rpm for 5 seconds at 4°C. After the supernatant was removed, the remaining product was dried under reduced pressure. 10 µL of 10x nuclease S1 buffer was added thereto, and digestion into a monomer was carried out with 1 µL of 10x nuclease S1. Then, 11 µL of the digested solution and 40 µL of ammonium acetate solution were taken and the mixture was analyzed by HPLC in the same manner as in Example 1, whereby a similar result to that of Example 1 was obtained. The yield was 5%.

### Example 10 (Aminoacylation using dendrimer having a cationic group on the surface)

### <Composition of reaction solution for aminoacylation>

| | |
|---|---|
| Solution A: 0.45 mM dendrimer/100 mM imidazole buffer (G3-C6NMe 31.2 mg, G3-C11NMe 31.4 mg, G4-C6NMe 32.4 mg and G4-C11NMe 32.8 mg were added to 100 mM imidazole buffer and dissolved respectively.) | 18 µL |
| Solution B: 100 mM Pentenoyl-napAla-OCM/DMF | 1 µL |
| Solution C: 0.2 mM tRNA/H₂O | 1 µL |
| | 20 µL |

The foregoing three solutions A, B and C were mixed in a microtube and mixed with a vortex mixer (ultrasonic wave) at room temperature for 5 minutes. Then, 60 µL of 1.5 M AcOK was added to the reaction solution and the reaction was terminated. 80 µL of phenol/chloroform was added thereto, and extraction was carried out. The supernatant was recovered and 60 µL of chloroform was added and extraction was carried out. Then, 360 µL of ethanol was added and the mixture was let stand at -30°C for 1 hour. Then, the mixture was centrifuged at 15,000 ppm for 30 minutes at 4°C and the supernatant was removed. Then, 70% ethanol (-30°C) was added and the mixture was centrifuged at 15,000 ppm for 5 seconds at 4°C. After the supernatant was removed, the remaining product was dried under reduced pressure for 15 minutes and the target product was obtained. The yield was 21%.

To the product, 10 µL of 10x nuclease S buffer and 1 µL of 10x nuclease S1 were added, and S1 digestion was carried out by incubating the mixture at 37°C for 15 minutes.

Ammonium acetate solution was added to the digested solution to make the total volume 50 µL and this was analyzed by HPLC in the same manner as in Example 1 and a similar result to that of Example 1 was obtained.

### Example 11 (Aminoacylation using Pentenoyl-napAla-OSu)

Aminoacylation of a tRNA was carried out in the same manner as in Example 3 except for using succinimide ester (OSu) instead of cyanomethylester (OCM) as an active ester.

### <Composition of reaction solution for aminoacylation>

| | |
|---|---|
| 20 mM CTACl/100 mM imidazole (pH7.5) | 18 µL |
| 100 mM Pentenoyl-2-napAla-OSu/DMF | 1 µL |
| 200 µM tRNA | 1 µL |
| | 20 µL |

After the foregoing samples were mixed and the mixture was mixed by ultrasonic wave and reacted with each other in the same manner as in Example 3, a post-treatment was carried out in the same manner as in Example 3 and an HPLC analysis was carried out in the same manner as in Example 1, whereby a similar result to that of Example 1 was obtained. The yield was 30%.

### Industrial Applicability

As the drawbacks of the conventional aminoacylation methods, the main points are 1) only an amino acid with a structure similar to that of a natural amino acid can be introduced, and 2) an artificial aminoacyl synthetase corresponding to an amino acid to be introduced needs to be prepared in each case, and further enormous efforts are required in order to prepare it.

On the contrary, according to the method of the present invention, it is not necessary to prepare or use an artificial aminoacyl synthetase corresponding to each amino acid, and a desired amino acid can be introduced in a tRNA by using organic chemical means. Therefore, any amino acid such as a nonnatural amino acid which could not be bound to a tRNA with a conventional aminoacyl-tRNA synthetase, for example, a nonnatural amino acid with a structure that is significantly different from that of a natural amino acid, an amino acid with a fluorescent property, an amino acid with a photoisomerization ability, an amino acid with an oxdating ability, an amino acid with a medicinal effect against HIV virus and the like can be effectively aminoacylated.

If an aminoacylated tRNA can be easily obtained, practical application of a protein in which a nonnatural amino acid has been introduced will go forward. In this case, by using the technique in the method for mutating DNA by random insertion and deletion for which the present inventors have applied for a patent before (specification of JP-A-2001-57478) or the technique in the four-base codon method which the present inventors have published previously [Appl Microbiol Biotechnol (2001) 57: 274-281], synthesis of a useful protein can be carried out more effectively.

## Claims

1. A method for aminoacylating a tRNA **characterized in that**, in the production of an aminoacyl-tRNA by selectively aminoacylating a tRNA, the tRNA and an amino acid are brought close to each other to react with each other.

2. The aminoacylation method according to claim 1, wherein the tRNA and the amino acid are enclosed in the vicinity of the micelle-water interface and brought close to each other to react with each other.

3. The aminoacylation method according to claim 2, wherein the carboxyl group of the amino acid is activated and enclosed in the micelle, and only the hydroxyl group region at the 3' end of the tRNA is inserted in the vicinity of the micelle-water interface, whereby the hydroxyl group at the 3' end and the activated carboxyl group are brought close to each other in the vicinity of the micelle-water interface to react with each other.

4. The aminoacylation method according to claim 2, wherein the carboxyl group of the amino acid is activated inside the micelle by using a condensing agent, and only the hydroxyl group region at the 3' end of the tRNA is inserted in the vicinity of the micelle-water interface, whereby the hydroxyl group at the 3'end and the activated carboxyl group are brought close to each other in the vicinity of the micelle-water interface to react with each other.

5. The aminoacylation method according to claim 3 or 4, wherein the 3' end group region is rendered hydrophobic by site-specifically and complementarily binding a peptide nucleic acid in which a hydrophobic functional group has been introduced at the end thereof to the tRNA in such a manner that the hydrophobic group comes close to the vicinity of the 3' end of the tRNA, whereby only the hydroxyl group region at the 3' end of the tRNA is inserted in the vicinity of the micelle-water interface.

6. The aminoacylation method according to any one of claims 2 to 5, wherein an amino acid whose amino group has been protected is used.

7. The aminoacylation method according to any one of claims 2 to 6, wherein the reaction is carried out in the presence of a surfactant.

8. The aminoacylation method according to any one of claims 2 to 6, wherein the reaction is carried out in the presence of polyethyleneimine or a dendrimer with a cationic group on a surface thereof.

9. The aminoacylation method according to any one of claims 2 to 8, wherein the reaction is carried out in an O/W (oil in water) type micelle.

10. The aminoacylation method according to any one of claims 2 to 8, wherein the reaction is carried out in an oil free system.

11. The aminoacylation method according to any one of claims 2 to 10, wherein the reaction is carried out by using a transesterification catalyst exhibiting a high catalytic activity at around a neutral pH.

12. The aminoacylation method according to claim 1, wherein a peptide nucleic acid specifically and complementarily binding to the tRNA is interposed as an antisense molecule, whereby the tRNA and the amino acid are brought close to each other to react with each other.

13. The aminoacylation method according to claim 12, wherein the amino acid is bound to the antisense molecule through an ester bond in advance and reacted with the tRNA.

14. The aminoacylation method according to claim 13, wherein the reaction is carried out by using the one in which the amino acid has been bound to the antisense molecule through an active ester.

15. The aminoacylation method according to claim 14, wherein the reaction is carried out by using the one in which a linker has been provided between the antisense molecule and the active ester.

16. The aminoacylation method according to any one of claims 13 to 15, wherein the one in which a cationic amino acid has been introduced at the other end of the antisense molecule is used.

17. The aminoacylation method according to claim 12, wherein the reaction with the tRNA is carried out by using a compound represented by the following formula [1]:
H-cAm-PNA-L-E-Am [1]
[wherein -cAm- represents a cationic amino acid residue or an oligopeptide residue consisted of 2 to 5 cationic amino acids, -PNA- represents a peptide nucleic acid residue, -L- represents a linker, -E- represents an active ester residue, and -Am represents an amino acid residue to be introduced in the tRNA].

18. The aminoacylation method according to any one of claims 12 to 17, wherein the reaction is carried out by using a transesterification catalyst exhibiting a high catalytic activity at around a neutral pH.

19. The aminoacylation method according to any one of claims 12 to 18, wherein a reaction terminator is used.

20. The aminoacylation method according to claim 19, wherein the reaction terminator is a peptide nucleic acid which forms a complementary pair with the peptide nucleic acid specifically and complementarily binding to the tRNA.

21. The aminoacylation method according to any one of claims 12 to 18, wherein the reaction is carried out by using further DNA as the antisense molecule other than the peptide nucleic acid which specifically and complementarily binds to the tRNA.

22. The aminoacylation method according to claim 21, wherein the reaction is terminated by raising the temperature of a reaction system.

23. The aminoacylation method according to claim 22, wherein the reaction is terminated by raising the temperature of the reaction system to 25°C.

24. A compound represented by the formula [1]:
H-cAm-PNA-L-E-Am [1]
[wherein -cAm- represents a cationic amino acid residue or an oligopeptide residue consisted of 2 to 5 cationic amino acids, -PNA- represents a peptide nucleic acid residue, -L- represents a linker, -E- represents an active ester residue, and -Am represents an amino acid residue to be introduced in the tRNA].

25. The compound according to claim 24, wherein, in the formula [1], -cAm- is -LysLys-, -PNA- is -CGTGGT-, and -Am is a nonnatural amino acid.


SEQUENCE LISTING

<110> Japan Science and Technology Agency

<120> Method of aminoacylating tRNA

<130> JA826102

<150> JP2002-262301
<151> 2002-09-09

<160> 1

<170> PatentIn version 3.1

<210> 1
<211> 77
<212> RNA
<213> Yeast

<220>
<221>
<223> tRNA (Phe CGGG)

<400> 1
gcggauuuag cucaguuggg agagcgccag acucccgaau cuggaggucc uguguucgau 60

ccacagaauu cgcacca 77